# EUROPEAN PATENT APPLICATION

(11) **EP 4 079 845 A1**
(43) Date of publication of application: **26.10.2022**
(21) Application number: 20903722.5
(22) Date of filing: 21.12.2020
(51) Int. Cl.: C12N 9/00, C07K 14/565, C12N 9/48, C12N 15/70, C12P 21/00

(54) **METHOD FOR ENHANCING WATER SOLUBILITY OF TARGET PROTEIN BY WHEP DOMAIN FUSION**

(30) Priority: 19.12.2019 KR 20190171057
(71) Applicant: Industry-Academic Cooperation Foundation, Yonsei University, Seoul 03722 (KR)
(72) Inventor: SEONG, Baik Lin, Seoul 03722 (KR); LEE, Jin Hee, Seoul 03722 (KR); KIM, Young Seok, Seoul 03722 (KR); CHEONG, Yu Cheol, Seoul 03722 (KR)
(74) Representative: Holt, Lucy Rose
(86) International application number: PCT/KR2020/018837
(87) International publication number: WO 2021/125917

(57) **Abstract**

The present invention relates to a fusion protein for enhancing expression efficiency of a target protein. More specifically, the present invention relates to a glutamyl-prolyl-tRNA synthetase from human (hEPRS) WHEP domain (including WHEP domains TRS-1, TRS-2, and TRS-3 which locate at middle sites of the EPRS protein, and linkers connecting the three domains therethrough). When used as a fusion protein for expressing a target protein in E. coli, the hEPRS WHEP domain according to the present invention enhanced water solubility of the target protein.

## Description

### [Technical Field]

The present invention relates to a fusion protein for increasing the expression efficiency of a target protein. More particularly, the present invention relates to WHEP domains (including WHEP domains TRS-1, TRS-2 and TRS-3 located in the central area of an EPRS protein and linkers connecting the three domains) of human glutamyl-prolyl-tRNA synthetase (hEPRS). When the WHEP domains of hEPRS according to the present invention are used as fusion proteins for expression of a target protein in *E. coli,* the solubility of the target protein is improved.

### [Background Art]

Generally, proteins produced by biotechnology may broadly be classified into pharmaceutical and research proteins such as immunomodulators, enzyme inhibitors, or hormones and industrial proteins such as diagnostic proteins or enzymes added for a specific reaction, and production process technology development and industrialization are being promoted mainly using these two types of proteins. Particularly, when a useful recombinant protein is produced using recombinant microorganism technology, because of its advantages such as well-known genetic information, construction of various vector systems, and rapid culture in a relatively cheap medium at a high concentration, *E. coli* is used for various purposes in research or industry.

In the production of recombinant proteins in *E. coli,* various expression vectors with a strong inducible promoter have been developed and used for the production of foreign proteins. However, in the case of using *E. coli* as a host cell, a protein to be manufactured may be frequently degraded by a proteolytic enzyme in *E. coli* to decrease yield, and particularly, it is known that this tendency is more pronounced in the expression of a polypeptide with a small molecular weight of 10 kDa or less. In addition, generally, since transcription and translation of a protein occur in *E. coli* at almost the same time, an insoluble aggregate (inclusion body) is frequently found in the overexpression of a recombinant protein, and in the case of polypeptides expressed as an aggregate, a folding intermediate may be non-selectively bound to other protein impurities (a chaperone, a ribosome, an initiation factor, etc.) in host cells by an intermolecular disulfide bond or hydrophobic interaction, thereby having a disadvantage of decreasing purity in the aggregate of a target polypeptide. In addition, to make the protein expressed as described above an active form, the protein has to undergo a refolding process in which the protein is dissolved using a denaturant such as guanidine hydrochloride or urea, and then diluted, but in this case, there is the problem of a decreased production yield in that the protein is not folded into an active form (Marston FA et al., Biochem J 240(1):1-12, 1986).

The inventors conducted research to confirm the solubility-enhancing ability of a WHEP domain as a fusion protein. Preferentially, TRS-1 and TRS-2 having only one WHEP domain, and multiple WHEP domains (referred to as EPRS) including three WHEP domains (TRS-1, TRS-2 and TRS-3) and linkers were manufactured. In addition, the ability of the three types of fusion proteins to enhance target protein solubility was confirmed. It was confirmed that the solubility-enhancing ability of the multiple WHEP domain among the three fusion proteins is most superior, and the present invention was completed.

To express conventionally known proteins that are soluble and difficult to express in E *coli,* solubility was compared using TRS-1, TRS-2 or EPRS as a fusion protein and a green fluorescent protein (GFP) as a target protein. As a result, it was confirmed that, among the fusion proteins including the WHEP domains of hEPRS, EPRS most highly contributes to an increase in solubility. The solubility-enhancing effect caused by EPRS was additionally confirmed with other target proteins, such as interferon-beta (IFN-β), TEV protease, and heat-labile enterotoxin subunit B (LTB).

### [Disclosure]

### [Technical Problem]

The present invention is directed to providing a peptide capable of enhancing the solubility and folding of a target protein using a WHEP domain as a fusion partner, and an expression vector or gene construct for producing a recombinant protein.

The present invention is also directed to providing a configuration of a WHEP domain capable of significantly increasing the solubility of a target protein.

The present invention is also directed to providing a recombinant microorganism which is transformed with the expression vector or into which a gene construct is inserted, and a method of producing a recombinant protein using the same.

### [Technical Solution]

The present invention provides a peptide for enhancing the expression efficiency of a target protein, which includes the sequence of a domain isolated from human glutamyl-prolyl tRNA synthetase (hEPRS).

According to one exemplary embodiment of the present invention, the domain may include TRS-1 (SEQ ID NO: 1) and TRS-2 (SEQ ID NO: 2), and most preferably, the domain includes TRS-1 (SEQ ID NO: 1) and TRS-2 (SEQ ID NO: 2).

The peptide of the present invention may further include a linker sequence to connect the sequences of the domains with each other, and the linker sequence may be represented by SEQ ID NO: 3.

In addition, the peptide may further include a tag sequence for enhancing the expression efficiency of a target protein.

In the present invention, the target protein may be one or more selected from the group consisting of an antigen, an antibody, a cell receptor, an enzyme, a structural protein, serum and a cell protein, and the antigen may be one or more selected from the group consisting of novel coronavirus (SARS-CoV-2), the hemagglutinin (HA) of influenza virus, respiratory syncytial virus (RSV) and Nipah virus. According to the most exemplary embodiment of the present invention, the antigen is RSV.

The present invention also provides a polynucleotide encoding the peptide.

According to one embodiment of the present invention, an expression vector which includes a polynucleotide encoding a target protein; and a polynucleotide encoding a peptide according to any one of claims 1 to 8 binding to the 5'-end of the polynucleotide encoding the target protein is provided.

According to another embodiment of the present invention, host cells transformed with the expression vector are provided, and the host cells may be *E. coli.*

The present invention provides a method of producing a soluble target protein, which includes: (A) preparing an expression vector including a polynucleotide encoding a target protein and a nucleotide encoding the peptide of any one of claims 1 to 8 binding to the 5' end of the polynucleotide; (B) preparing a transformant by introducing the expression vector into host cells; and (C) culturing the transformant to induce the expression of a recombinant target protein and obtaining the target protein.

### [Advantageous Effects]

A method of producing a recombinant protein using a WHEP domain according to the present invention as a fusion partner improves the solubility and expression rate of a target protein, and is useful for developing and producing various target proteins for medical and industrial purposes.

### [Description of Drawings]

FIG. 1 shows an expression plasmid designed to use TRS-1, TRS-2 and EPRS as fusion proteins, in which a target protein is inserted into a multiple cloning site (MCS), and a histidine tag (6X His) for purification and a TEV protease recognition site (TEV site) for cleaving the fusion proteins and the target protein are included.
FIG. 2 is an image showing the improvement in soluble expression and the activity of GFP, caused by EPRS.
FIG. 3 is an image showing the improvement in soluble expression and the activity of interferon-beta (IFN-β), caused by EPRS.
FIG. 4 is an image showing the improvement in soluble expression and the activity of TEV protease, caused by EPRS.

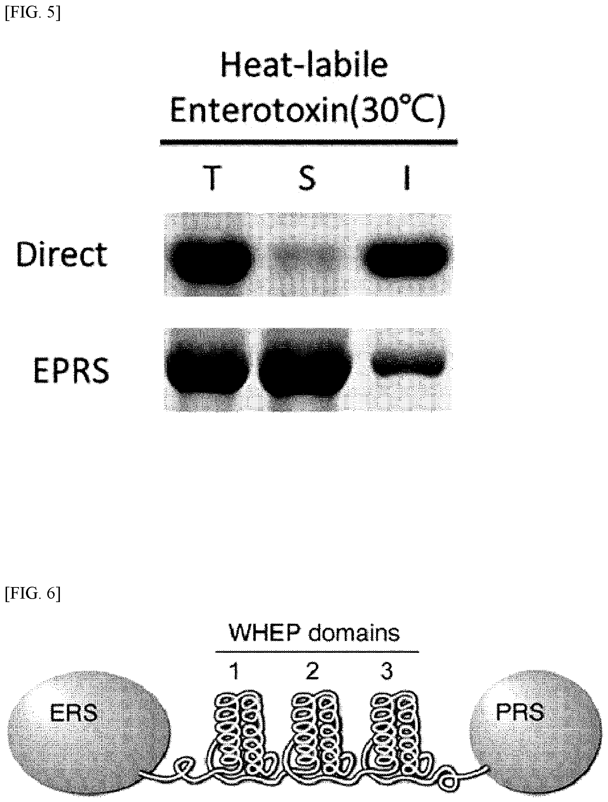

FIG. 5 is an image confirming the improvement in soluble expression of a multimeric protein, caused by EPRS. In addition, an effect of increasing the expression rate of a soluble protein, caused by EPRS, was further confirmed by expressing the multimeric protein in *E. coli.* Heat-labile enterotoxin is a protein constituting a pentamer, and also known to be difficult to express in a soluble form in *E. coli.* As a result of confirming the expression of the protein fused with EPRS, it was confirmed that the EPRS-fused form showed a much higher soluble expression rate, compared to a direct form.
FIG. 6 is a schematic diagram showing a fused form of WHEP domains and ERS.
FIG. 7 is an image confirming the the expression of EPRS fused with RBD and S1 of COVID-19 according to one embodiment of the present invention.
FIG. 8 is an image confirming the expression of EPRS fused with RSV F-bacterioferritin according to one embodiment of the present invention.
FIG. 9 is an image confirming the expression of EPRS fused with LTB-JEVED3 according to one embodiment of the present invention.
FIG. 10 show the result of size-exclusion chromatography for TEV protease-treated EPRS-LTB.
FIG. 11 shows the result of size-exclusion chromatography for EPRS-LTB-JEVED3.
FIG. 12 shows the result of size-exclusion chromatography for TEV protease-treated EPRS-LTB-JEVED3.

### [Modes of the Invention]

The inventors confirmed that, among the fusion proteins including WHEP domains of hEPRS, EPRS most greatly contributes to the increase in solubility, and the present invention was completed.

The present invention provides a peptide capable of improving the solubility and folding of a target protein using a WHEP domain as a fusion partner, and an expression vector or gene construct for producing a recombinant protein.

The "vector" used herein refers to DNA that can introduce a target DNA fragment into host bacteria to proliferate in a DNA recombination experiment and is also referred to as a cloning vehicle. The vector DNA undergoes ring opening by cleavage with a restriction enzyme, and a target DNA fragment is introduced into the host bacteria by insertion and linkage to the vector DNA. The vector DNA in which the target DNA fragment is linked is replicated as the host bacteria are proliferated, and distributed to each cystic cell along with bacterial division, thereby maintaining the target DNA fragment for generations, and the vector DNA may be, for example, a plasmid or a phage chromosome.

Methods such as the selection, construction and transformation of the vector, and the expression of a recombinant protein may be easily performed by those of ordinary skill in the art to which the present invention belongs, and some modifications in conventional methods are also included in the present invention.

The "transformation" used herein refers to a genetic change in the trait of an individual or cell by DNA, which is the genetic material given from the outside.

The delivery (introduction) of the vector into a host cell may use a delivery method well known in the art. As a delivery method, for example, a microinjection method, a calcium phosphate precipitation method, an electroporation method, a liposome-mediated transfection method, or a gene bombardment method may be used, but the present invention is not limited thereto. A chemical treatment method such as PEG or a gene gun may be used.

Culture conditions in the culture of a transformant may be appropriately selected and used depending on a host cell. Conditions such as a temperature, the pH of a medium and incubation time may be appropriately adjusted to be suitable for cell growth and mass production of a protein during culture.

The host cell in the present invention may be a host cell conventionally used in the art, and preferably, *E. coli.*

Hereinafter, embodiments of the present invention will be described with reference to the drawings. The advantages and features of the present invention and the methods of accomplishing the same may be clearly understood with reference to the detailed description of exemplary embodiments and the accompanying drawings. However, the present invention is not limited to the exemplary embodiments disclosed below, and may be embodied in many different forms. These exemplary embodiments are merely provided to complete the disclosure of the present invention and fully convey the scope of the present invention to those of ordinary skill in the art, and the present invention should be defined by only the accompanying claims. Throughout the specification, like reference numerals denote like elements.

Unless defined otherwise, all terms (including technical and scientific terms) used herein may be used in a sense that is commonly understood by one of ordinary skill in the art to which the present invention belongs. Also, generally used predefined terms are not ideally or excessively interpreted unless explicitly defined otherwise. The terms used herein are for describing embodiments and are not intended to limit the present invention. Herein, singular forms include plural forms unless specifically stated otherwise.

### [Example 1]

### Example 1-1. Construction of protein expression vector

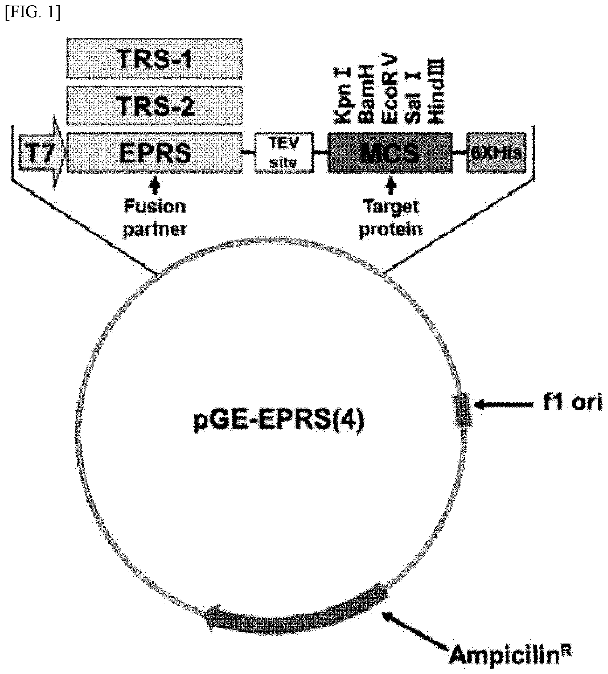

As a protein expression vector, a pGE-LysRS expression vector was used (Choi SI et al., Protein solubility and folding enhancement by interaction with RNA, PLoS ONE (2008), 3: e2677). A pGE-TRS-1, pGE-TRS-2, or pGE-EPRS expression vector was manufactured by adjusting the expression of pGE-LysRS by a T7 promoter, cleaving the LysRS gene at one of cleavage sites in Ndel and MCS (Kpn1-BamH1-EcoRV-Sal1-Hind3), and inserting TRS-1, TRS-2, or EPRS into the site. In addition, a TRS-1, TRS-2 or EPRS-fused target protein expression vector was manufactured by inserting a target protein into a C-terminus site of each expression vector (FIG. 1). As the target protein, GFP, IFN-β, TEV protease or LTB was used. The expression vector manufactured as described above is pGE-GFP, pGE-TRS-1-GFP, pGE-TRS-2-GFP, pGE-EPRS-GFP, pGE-IFN-β, pGE-EPRS-IFN-β, pGE-Tev, pGE-EPRS-Tev, pGE-LTB, or pGE-EPRS-LTB.

### Example 1-2. Protein expression and confirmation of purification pattern through SDS-PAGE

The manufactured protein expression vector was transformed into BL21(DE3)-pLysS or SHuffle^{®} T7 competent cells and cultured. All transformed *E. coli* were cultured in an LB medium containing 50 µg/mL of ampicillin, pLysS-containing *E. coli* was cultured in a medium supplemented with 34 µg/mL of chloramphenicol. A culture temperature is different for each protein, and culture was performed at 16 to 37 °C. When the OD600 value *of E. coli* became 0.5 or more, 0 µM to 1 mM of IPTG was added to activate the T7 promoter, and in order to sufficiently produce a protein, after the IPTG addition, the *E. coli* was cultured at 16 to 37 °C for approximately 3 hours, at 25 °C for approximately 5 hours, and 16 to 24 °C for approximately 16 hours. The sufficiently cultured El coli was centrifuged to remove a supernatant, and then stored. Afterward, 0.3 mL of PBS was added to harvested *E. coli* corresponding to 5 mL of the LB medium for ultrasonication, thereby obtaining a lysate. In addition, the lysate was centrifuged and divided into a soluble fraction and a pellet fraction, and each of the total lysate, the soluble fraction and the pellet fraction was analyzed by SDS-PAGE.

### Example 1-3. Protein expression and purification

Each of the manufactured pGE-GFP, pGE-TRS-1-GFP, pGE-TRS-2-GFP, pGE-EPRS-GFP, pGE-IFN-β, pGE-EPRS-IFN-β, pGE-Tev, and pGE-EPRS-Tev expression vectors wwas transformed into BL21(DE3)-pLysS competent cells and cultured. All transformed E. *coli* were cultured in LB containing 50 µg/mL of ampicillin and 34 µg/mL of chloramphenicol. When the OD600 value *of E. coli* became 0.5 or more, 1 mM IPTG was added to activate a T7 promoter, and in order to sufficiently produce a protein, after the IPTG addition, the *E. coli* was cultured at 30 °C for approximately 6 hours. The sufficiently cultured *E. coli* was centrifuged to remove a supernatant, and stored.

PBS was added to the harvested *E. coli* for centrifugation, thereby obtaining a lysate. In addition, the lysate was centrifuged to obtain only a soluble fraction and then purified by Ni²⁺ chromatography, and the purified protein was subjected to dialysis in PBS and concentrated using Centriprep.

### Example 1-4. Measurement of GFP activity

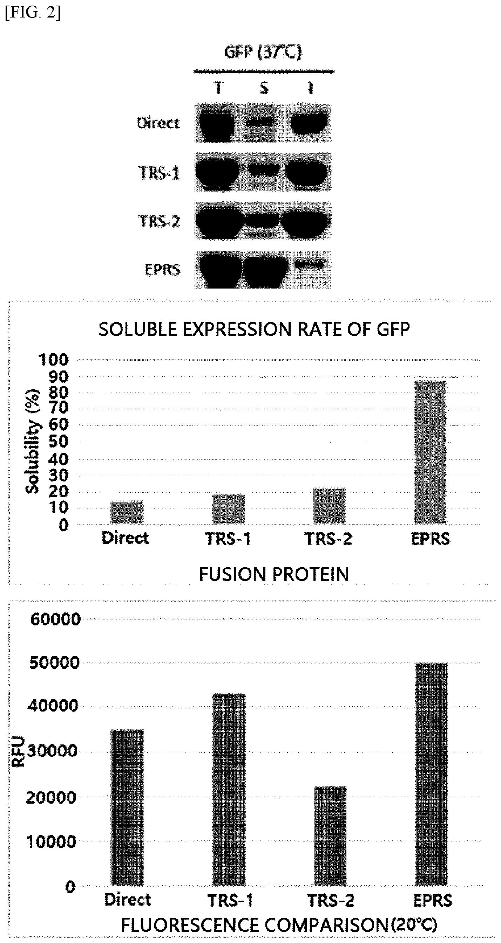

An experiment was performed to measure the activity of a GFP protein produced per unit cell. After the completion of protein expression, PBS was added to a *E. coli* culture from which a supernatant was removed through centrifugation, and the resulting solution was subjected to ultrasonication, thereby obtaining a lysate. Afterward, the lysate was centrifuged to obtain only a soluble fraction, and then the actual activity of GFP was measured by measuring fluorescence with a spectrophotometer (FIG. 2).

A green fluorescent protein (GFP) is known as a protein that is expressed in an insoluble form when expressed alone in *E. coli.* As a result of confirming the expression pattern after direct GFP expressed alone without a fusion protein and TRS-1-, TRS-2-, and EPRS-fused fusion proteinswere expressed in *E. coli,* all of the fusion proteins showed an increase in soluble expression rate compared to the direct GFP, and it was confirmed that, particularly, the soluble expression rate of the EPRS-fused GFP was very highly increased.

As a result of measuring the activity of expressed GFP per culture dose, the TRS-2-fused GFP showed fluorescence activity similar to direct GFP, and TRS-1 and EPRS-fused GFPs showed higher fluorescent activity.

Therefore, it was confirmed that the soluble expression level of the target protein GFP is increased by TRS-1, TRS-2 and EPRS, and the produced proteins were active.

### Example 1-5. Measurement of IFN-beta activity

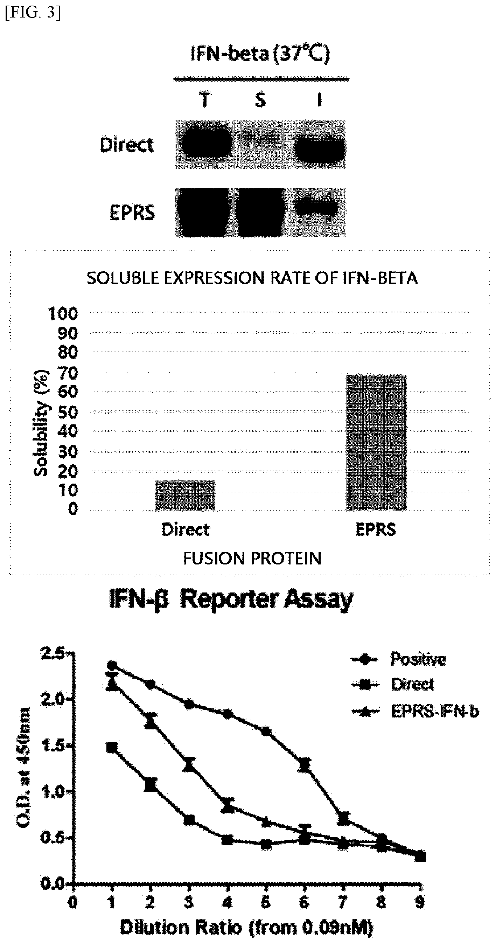

The activity of IFN-beta expressed in *E. coli* was measured using HEK-Blue^{™} IFN-α/β cells (InvivoGen). The experiment was conducted according to the seller's protocol, and it is briefly summarized as follows. 180 µL of a suspension of HEK-Blue^{™} IFN-α/β cells modified to observe a signaling pathway by IFN-beta and 20 µL of an IFN-beta sample were added to a 96-well plate, and incubated in an incubator (37 °C, 5% CO₂) overnight. The next day, 20 µL of a supernatant in which a reaction occurred in HEK-Blue^{™} IFN-α/β cells and 180 µL of a QuantiBlue^{™} solution were mixed, and reacted at 37 °C for approximately 30 minutes to 3 hours. After the reaction was completed, a level was measured at a wavelength of 620 to 655 nm with a spectrophotometer (FIG. 3).

Interferon-beta (IFN-β) is also a protein that is expressed insoluble in *E. coli,* and direct IFN-β having no fusion protein shows a characteristic of a very low soluble expression rate in E. *coli.* When EPRS was used as a fusion protein, the fusion protein showed a high soluble expression rate.

When the activity of purified IFN-β was measured with HEK-Blue^{™} IFN-α/β cells (InvivoGen), the activity of commercial IFN-β expressed and purified in CHO cells was the highest, and the activity of the IFN-β fusion protein in which EPRS was fused was the next highest. Direct IFN-β showed the lowest activity.

Therefore, it was confirmed that the expression of soluble and active-form IFN-β was promoted by EPRS.

### Example 1-6. Measurement of Tev protease activity

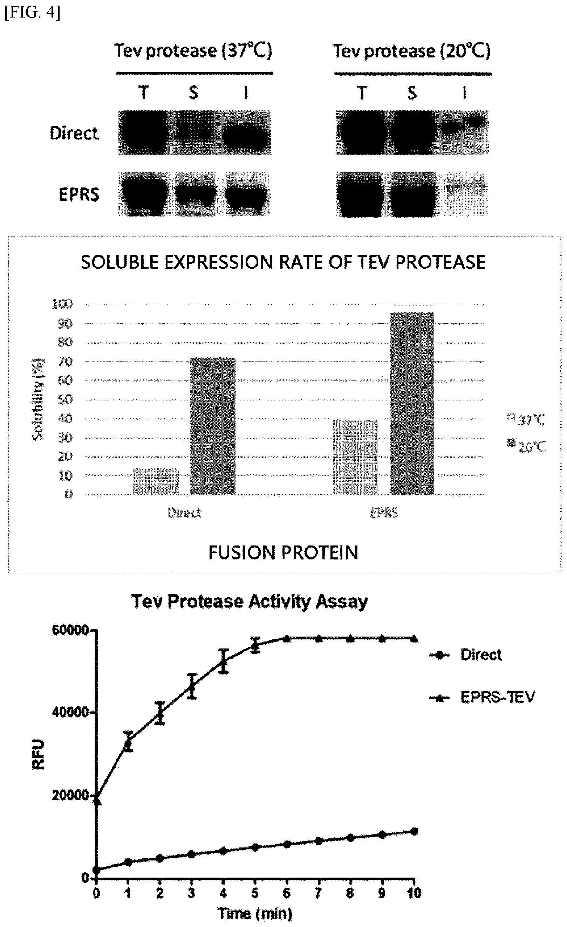

To measure the activity of TEV protease expressed in *E. coli,* a SensoLyte ^{®} 520 TEV Activity Assay Kit (^{∗}Fluorimetric^{∗} kit) was used. The experiment was conducted according to the seller's protocol, and simply summarized as follows. The TEV protease and 5-FAM/QXL^{®} 520 TEV substrate for experimentation were put into a 96-well plate and fluorescence was measured every minute. Here, the measured fluorescence was Ex/Em = 490/520 nm. As the substrate is cleaved by the TEV protease, fluorescence increased, and using this, the activity of the TEV protease was measured (FIG. 4).

Tobacco Etch Virus (TEV) protease is a protein with high commercial demand, and a type of protein difficult to be obtained as a recombinant protein in *E. coli.* When there was no fusion protein, direct TEV protease showed a low soluble expression rate in *E. coli,* and when EPRS was fused, an effect of increasing the soluble expression rate was shown. When an expression temperature was lowered (20 °C) in *E. coli* culture, both of the direct TEV protease and the EPRS-TEV protease showed an increased soluble expression rate, and particularly, the EPRS-TEV protease showed a soluble expression rate of 90% or more.

As a result of confirming the activity of purified TEV proteases with a SensoLyte^{®} 520 TEV Activity Assay Kit, even with the same amount, the direct TEV protease showed lower activity, and the structure showing the steepest enzymatic activity was EPRS-fused EPRS-TEV.

Therefore, the soluble and active-form expression of TEV protease, caused by EPRS, was confirmed.

### [Example 2]

### Example 2-1. Protein expression and confirmation of solubility by SDS-PAGE

Vectors manufactured using a receptor-binding domain (RBD) and relatively large spike subunit 1 (S1) of SARS-CoV-2 (COVID-19) as target proteins are pGE-EPRS-RBD and pGE-EPRS-S 1.

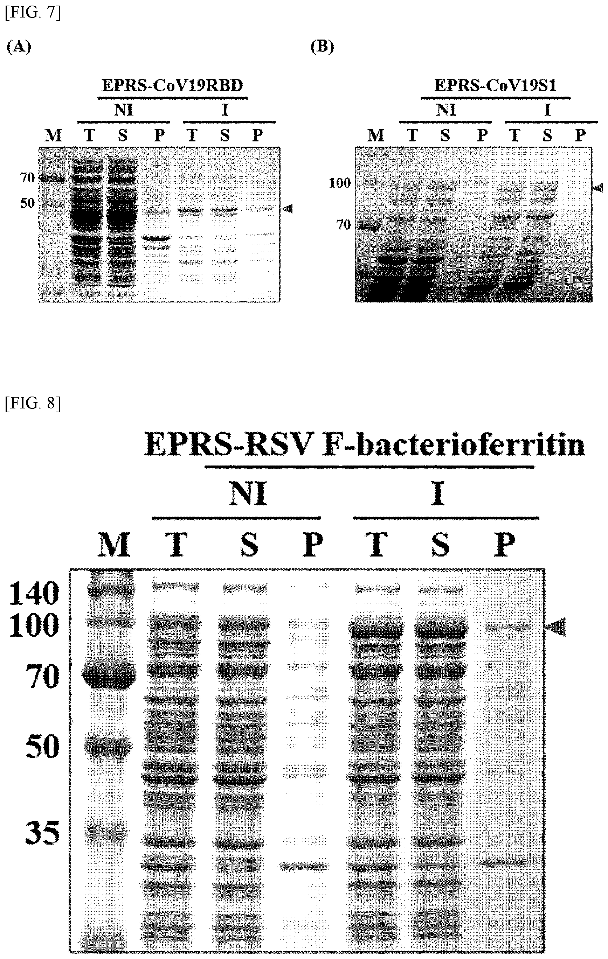

Each of the manufactured protein expression vectors was transformed into BL21 or HMS174 competent cells and cultured. All transformed *E. coli* were cultured in an LB medium containing 50 µg/mL of ampicillin. When the OD600 value of the *E. coli* became 0.5 or more, to activate a T7 promoter, 0.5 mM IPTG was added, and then incubated at 16 °C for 16 hours. The cultured *E. coli* was centrifuged to remove a supernatant, and stored at -80 °C. Afterward, 0.3 mL of lysis buffer [50 mM Tris-Cl (pH 7.5), 300 mM NaCl, 10% glycerol, 2 mM betamercaptoethanol, 0.1% Tween-20] was added to the harvested *E. coli* corresponding to 5 mL of the LB medium and subjected to ultrasonication, thereby obtaining a lysate. In addition, the lysate was centrifuged and divided into a soluble fraction and a pellet fraction, and SDS-PAGE was used to analyze the total lysate, the soluble fraction, and the pellet fraction. The sizes of RBD and S1 were 27 kDa and 73 kDa, respectively, and the sizes of EPRS-fused RBD and S1 were 49 kDa and 95 kDa, respectively (see FIG. 7).

A vector manufactured using a respiratory syncytial virus (RSV) fusion (F) protein as a target protein and bacterioferritin as a scaffold protein for nanoparticles is pGE-EPRS-RSV F-bacterioferritin.

The manufactured protein expression vector was transformed into Shuffle T7 competent cells and cultured. All transformed *E. coli* were cultured in an LB medium containing 50 µg/mL of ampicillin. When the OD600 value of the *E. coli* is 0.5 or more, to activate a T7 promoter, 0.5 mM IPTG was added, and then incubated at 20 °C for approximately 6 hours. The cultured *E. coli* was centrifuged to remove a supernatant, and stored at -80 °C. Afterward, 0.3 mL of lysis buffer [50 mM Tris-Cl (pH7.5), 200 mM NaCl, 10% glycerol, 0.1% Tween-20] was added to the harvested *E. coli* corresponding to 5 mL of the LB medium and subjected to ultrasonication, thereby obtaining a lysate. In addition, the lysate was centrifuged and divided into a soluble fraction and a pellet fraction, and SDS-PAGE was used to analyze the total lysate, the soluble fraction, and the pellet fraction. The size of RSV F-bacterioferritin was 70 kDa, and the size of the EPRS-fused RSV F-bacterioferritin was 92 kDa (see FIG. 8).

A vector, which is manufactured with the domain III (ED3) of an envelope protein that is present in the form of a pentamer with 5 axes in the Japanese encephalitis virus structure and is known to play an important role in immune induction as a target protein and the heat-labile toxin B subunit (LTB) as a pentameric scaffold, is pGE-EPRS-LTB-JEVED3.

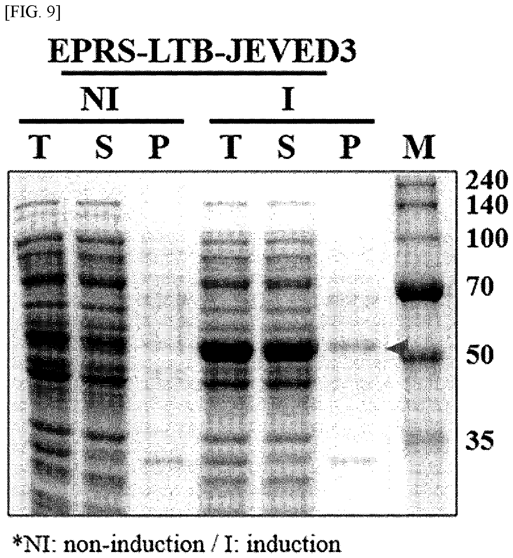

The manufactured protein expression vector was transformed into Shuffle T7 competent cells and cultured. All transformed *E. coli* were cultured in an LB medium containing 50 µg/mL of ampicillin. When the OD600 value of the *E. coli* became 0.5 or more, to activate a T7 promoter, 0.5 mM IPTG was added, and then incubated at 20 °C for approximately 6 hours. The cultured *E. coli* was centrifuged to remove a supernatant, and stored at -80 °C. Afterward, 0.3 mL of lysis buffer [50 mM Tris-Cl(pH7.5), 300 mM NaCl, 10% glycerol] was added to the harvested *E. coli* corresponding to 5 mL of the LB medium and subjected to ultrasonication, thereby obtaining a lysate. In addition, the lysate was centrifuged and divided into a soluble fraction and a pellet fraction, and SDS-PAGE was used to analyze the total lysate, the soluble fraction, and the pellet fraction. The size of LTB-JEVED3 is 24 kDa, and the size of EPRS-fused LTB-JEVED3 is 46 kDa (see FIG. 9).

### Example 2-2. Protein purification

The pGE-EPRS-LTB and pGE-EPRS-LTB-JEVED3 expression vectors transformed into Shuffle T7 competent cells were cultured in a LB medium containing 50 µg/mL of ampicillin. When the OD600 value of the *E. coli* became 0.5 or more, 1 mM of IPTG was added, and cultured at 20 °C for approximately 6 hours. The sufficiently cultured *E. coli* was centrifuged to remove a supernatant and stored at -80 °C.

A buffer [50 mM Tris-Cl (pH7.5), 300 mM NaCl, 10% glycerol, 5 mM imidazole] was added to the cultured *E. coli* and centrifuged, thereby obtaining a lysate. In addition, the lysate was centrifuged to obtain only a soluble fraction, and then the fraction was purified by nickel chromatography. The purified protein was dialyzed in a storage buffer [50 mM Tris-Cl (pH7.5), 300 mM NaCl, 0.1 mM EDTA], and concentrated using Amicon.

### Example 2-3. Confirmation of pentamer through size-exclusion chromatography

To verify whether the purified EPRS-LTB and EPRS-LTB-JEVED3 proteins form a pentameric structure, size-exclusion chromatography (SEC) using a Superdex 200 Increase 10/300 column (GE Healthcare) was performed, and the size of each protein was measured by calibration with a marker protein with a known size. Here, the composition of a buffer used herein is [50 mM Tris-Cl (pH7.5), 300 mM NaCl]. Both of an EPRS-fused protein and an EPRS-removed protein obtained by being treated with TEV protease to cleave a Tev cleavage site between EPRS and a target protein were verified. A fraction with a peak at the pentamer size was verified again by SDS-PAGE.

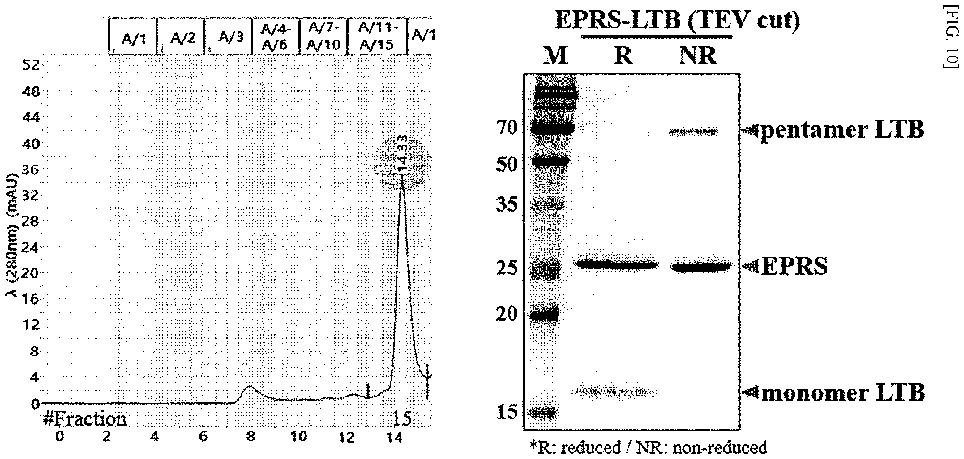

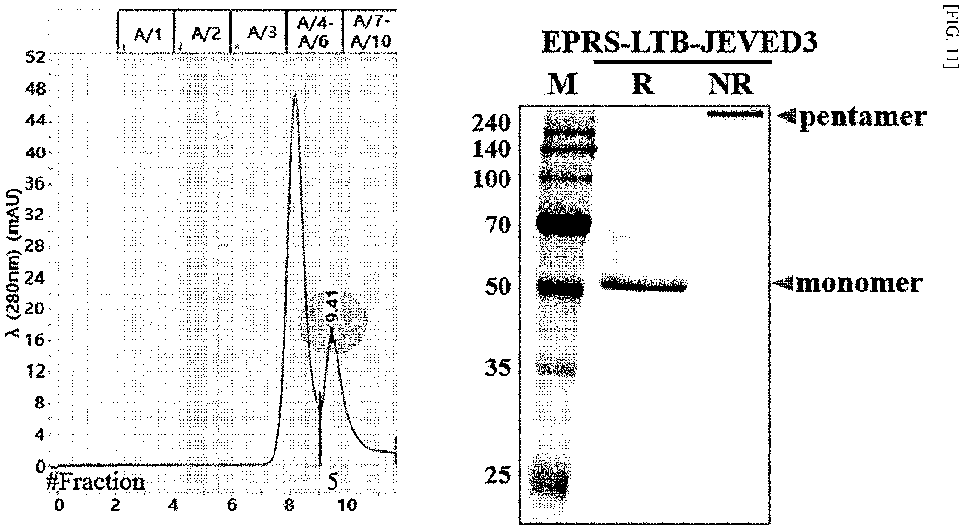

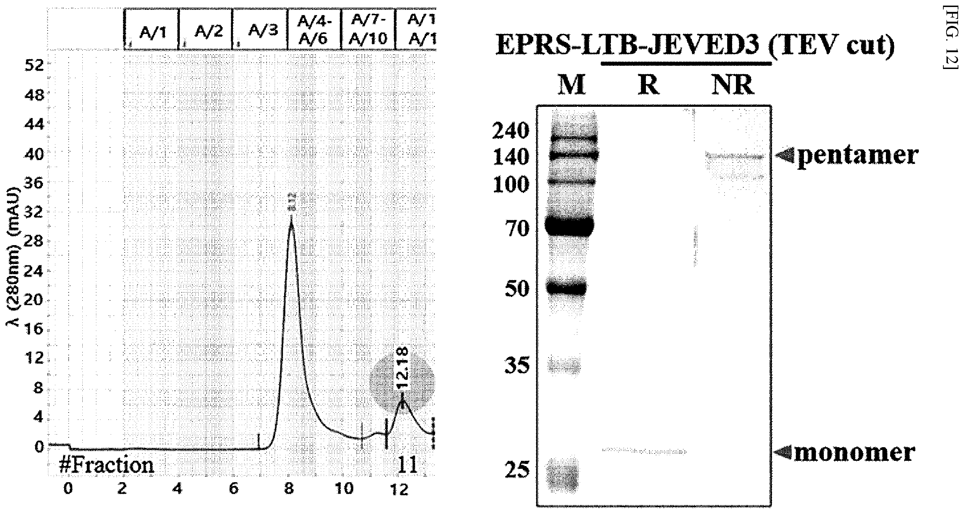

As a result, each protein showed a peak at an expected pentamer size (see FIGS. 10 to 12, left panel), and when the fraction of the peak was prepared in a reduced state with DTT and a non-reduced state without DTT and analyzed by SDS-PAGE, it was confirmed that bands of similar sizes are shown (see FIGS. 10 to 12, right panel). When the vectors were formed in a pentamer, the size of LTB is approximately 60 kDa, the size of EPRS-LTB-JEVED3 is approximately 230 kDa, and the size of LTB-JEVED3 is approximately 125 kDa. Therefore, it was confirmed that the fusion with EPRS cannot only increase the solubility of a target protein in *E. coli,* but also assembly into a multimer is possible by inducing suitable protein folding.
SEQ ID NO: 1 
TRS-1

LYNRVAVQGDVVRELKAKKAPKEDVDAAVKQLLSLKAEYKEKTGQEYKPGNP

P
SEQ ID NO: 2 
TRS-2

LYDEVAAQGEVVRKLKAEKSPKAKINEAVECLLSLKAQYKEKTGKEYIPGQPP
SEQ ID NO: 3 
TRS-3

LFDKVASQGEVVRKLKTEKAPKDQVDIAVQELLQLKAQYKSLIGVEYKP
SEQ ID NO: 4
   AEIGQNISSNSSASILESKS
SEQ ID NO: 5
   LSQSSDSSPTRNSEPAGLETPEAKV
SEQ ID NO: 6 
EPRS sequence

LYNRVAVQGDVVRELKAKKAPKEDVDAAVKQLLSLKAEYKEKTGQEYKPGNP

PAEIGQNISSNSSASILESKSLYDEVAAQGEVVRKLKAEKSPKAKINEAVECLLSLKAQY

KEKTGKEYIPGQPPLSQSSDSSPTRNSEPAGLETPEAKVLFDKVASQGEVVRKLKTEKAP

KDQVDIAVQELLQLKAQYKSLIGVEYKP
SEQ ID NO: 7 COVID-19 RBD 
FTVEKGIYQTSNFRVQPTESIVRFPNITNLCPFGEVFNATRFASVYAWNRKRISNC
VADYSVLYNSASFSTFKCYGVSPTKLNDLCFTNVYADSFVIRGDEVRQIAPGQTGKIAD
YNYKLPDDFTGCVIAWNSNNLDSKVGGNYNYLYRLFRKSNLKPFERDISTEIYQAGSTP
CNGVEGFNCYFPLQSYGFQPTNGVGYQPYRVVVLSFELLHAPATVCGPKKSTNLVKNK
CVNFNF
SEQ ID NO: 8 
COVID-19 S1

QCVNLTTRTQLPPAYTNSFTRGVYYPDKVFRSSVLHSTQDLFLPFFSNVTWFHAI

HVSGTNGTKRFDNPVLPFNDGVYFASTEKSNIIRGWIFGTTLDSKTQSLLIVNNATNVVI

KVCEFQFCNDPFLGVYYHKNNKSWMESEFRVYSSANNCTFEYVSQPFLMDLEGKQGNF

KNLREFVFKNIDGYFKIYSKHTPINLVRDLPQGFSALEPLVDLPIGINITRFQTLLALHRSY

LTPGDSSSGWTAGAAAYYVGYLQPRTFLLKYNENGTITDAVDCALDPLSETKCTLKSFT

VEKGIYQTSNFRVQPTESIVRFPNITNLCPFGEVFNATRFASVYAWNRKRISNCVADYSV

LYNSASFSTFKCYGVSPTKLNDLCFTNVYADSFVIRGDEVRQIAPGQTGKIADYNYKLPD

DFTGCVIAWNSNNLDSKVGGNYNYLYRLFRKSNLKPFERDISTEIYQAGSTPCNGVEGF

NCYFPLQSYGFQPTNGVGYQPYRVVVLSFELLHAPATVCGPKKSTNLVKNKCVNFNFN

GLTGTGVLTESNKKFLPFQQFGRDIADTTDAVRDPQTLEILDITPCSFGGVSVITPGTNTS

NQVAVLYQDVNCTEVPVAIHADQLTPTWRVYSTGSNVFQTRAGCLIGAEHVNNSYECD

IPIG
SEQ ID NO: 9
   Heat-labile toxin B subunit(LTB) 
APQTITELCSEYRNTQIYTINDKILSYTESMAGKREMVIITFKSGETFQVEVPGSQH
IDSQKKAIERMKDTLRITYLTETKIDKLCVWNNKTPNSIAAISMKN
SEQ ID NO: 10
   Bacterioferritin 
MKGDTKVINYLNKLLGNELVAINQYFLHARMFKNWGLKRLNDVEYHESIDEMK
HADRYIERILFLEGLPNLQDLGKLNIGEDVEEMLRSDLALELDGAKNLREAIGYADSVH
DYVSRDMMIEILRDEEGHIDWLETELDLIQKMGLQNYLQAQIREEG
SEQ ID NO: 11 
RSV F

QNITEEFYQSTCSAVSKGYLSALRTGWYTSVITIELSNIKENKCNGTDAKVKLIKQ

ELDKYKNAVTELQLLMQSTPPTNNRARRGGSGGSGGSGFLGFLLGVGSAIASGVAVCK

VLHLEGEVNKIKSALLSTNKAVVSLSNGVSVLTFKVLDLKNYIDKQLLPILNKQSCSISNI

ETVIEFQQKNNRLLEITREFSVNAGVTTPVSTYMLTNSELLSLINDMPITNDQKKLMSNN

VQIVRQQSYSIMCIIKEEVLAYVVQLPLYGVIDTPCWKLHTSPLCTTNTKEGSNICLTRTD

RGWYCDNAGSVSFFPQAETCKVQSNRVFCDTMNSLTLPSEINLCNVDIFNPKYDCKIMT

SKTDVSSSVITSLGAIVSCYGKTKCTASNKNRGIIKTFSNGCDYVSNKGMDTVSVGNTLY

YVNKQEGKSLYVKGEPIINFYDPLVFPSDEFDASISQVNEKINQSLAFIRKSDELL
SEQ ID NO: 12
   JEV Envelope domain III(ED3)

KGTTYGMCTEKFSFAKNPADTGHGTVVIELSYSGSDGPCKIPIVSVASLNDMTPV
GRLVTVNPFVATSSANSKVLVEMEPPFGDSYIVVGRGDKQINHHWHKAG

<110>    Industry-Academic Cooperation Foundation, Yonsei University

<120>    METHOD FOR ENHANCING SOLUBLE EXPRESSION OF TARGET PROTEINS BY
         USING FUSION PROTEIN OF WHEP DOMAIN

<130>    1068672

<150>    KR 10-2019-0171057
<151>    2019-12-19

<160>    12

<170>    KoPatentIn 3.0

<210>    1
<211>    53
<212>    PRT
<213>    Artificial Sequence

<220>
<223>    TRS1


<400>    1
Leu Tyr Asn Arg Val Ala Val Gln Gly Asp Val Val Arg Glu Leu Lys
  1               5                  10                  15

Ala Lys Lys Ala Pro Lys Glu Asp Val Asp Ala Ala Val Lys Gln Leu
             20                  25                  30

Leu Ser Leu Lys Ala Glu Tyr Lys Glu Lys Thr Gly Gln Glu Tyr Lys
         35                  40                  45

Pro Gly Asn Pro Pro
     50


<210>    2
<211>    53
<212>    PRT
<213>    Artificial Sequence

<220>
<223>    TRS2


<400>    2
Leu Tyr Asp Glu Val Ala Ala Gln Gly Glu Val Val Arg Lys Leu Lys
  1               5                  10                  15

Ala Glu Lys Ser Pro Lys Ala Lys Ile Asn Glu Ala Val Glu Cys Leu
             20                  25                  30

Leu Ser Leu Lys Ala Gln Tyr Lys Glu Lys Thr Gly Lys Glu Tyr Ile
         35                  40                  45

Pro Gly Gln Pro Pro
     50


<210>    3
<211>    49
<212>    PRT

<213>    Artificial Sequence

<220>
<223>    TRS3


<400>    3
Leu Phe Asp Lys Val Ala Ser Gln Gly Glu Val Val Arg Lys Leu Lys
1               5                   10                  15

Thr Glu Lys Ala Pro Lys Asp Gln Val Asp Ile Ala Val Gln Glu Leu
            20                  25                  30

Leu Gln Leu Lys Ala Gln Tyr Lys Ser Leu Ile Gly Val Glu Tyr Lys
        35                  40                  45

Pro




<210>    4
<211>    20
<212>    PRT
<213>    Artificial Sequence

<220>
<223>    Linker 1


<400>    4
Ala Glu Ile Gly Gln Asn Ile Ser Ser Asn Ser Ser Ala Ser Ile Leu
1               5                   10                  15

Glu Ser Lys Ser
            20


<210>    5
<211>    25
<212>    PRT
<213>    Artificial Sequence

<220>
<223>    Linker 2


<400>    5
Leu Ser Gln Ser Ser Asp Ser Ser Pro Thr Arg Asn Ser Glu Pro Ala
1               5                   10                  15

Gly Leu Glu Thr Pro Glu Ala Lys Val
            20                  25


<210>    6
<211>    200
<212>    PRT
<213>    Artificial Sequence

<220>
<223>    EPRS

<400>    6
Leu Tyr Asn Arg Val Ala Val Gln Gly Asp Val Val Arg Glu Leu Lys
1               5                   10                  15

Ala Lys Lys Ala Pro Lys Glu Asp Val Asp Ala Ala Val Lys Gln Leu
            20                  25                  30

Leu Ser Leu Lys Ala Glu Tyr Lys Glu Lys Thr Gly Gln Glu Tyr Lys
        35                  40                  45

Pro Gly Asn Pro Pro Ala Glu Ile Gly Gln Asn Ile Ser Ser Asn Ser
    50                  55                  60

Ser Ala Ser Ile Leu Glu Ser Lys Ser Leu Tyr Asp Glu Val Ala Ala
65                  70                  75                  80

Gln Gly Glu Val Val Arg Lys Leu Lys Ala Glu Lys Ser Pro Lys Ala
                85                  90                  95

Lys Ile Asn Glu Ala Val Glu Cys Leu Leu Ser Leu Lys Ala Gln Tyr
            100                 105                 110

Lys Glu Lys Thr Gly Lys Glu Tyr Ile Pro Gly Gln Pro Pro Leu Ser
        115                 120                 125

Gln Ser Ser Asp Ser Ser Pro Thr Arg Asn Ser Glu Pro Ala Gly Leu
    130                 135                 140

Glu Thr Pro Glu Ala Lys Val Leu Phe Asp Lys Val Ala Ser Gln Gly
145                 150                 155                 160

Glu Val Val Arg Lys Leu Lys Thr Glu Lys Ala Pro Lys Asp Gln Val
                165                 170                 175

Asp Ile Ala Val Gln Glu Leu Leu Gln Leu Lys Ala Gln Tyr Lys Ser
            180                 185                 190

Leu Ile Gly Val Glu Tyr Lys Pro
        195                 200


<210>    7
<211>    238
<212>    PRT
<213>    Artificial Sequence

<220>
<223>    COVID-19 RBD


<400>    7
Phe Thr Val Glu Lys Gly Ile Tyr Gln Thr Ser Asn Phe Arg Val Gln
1               5                   10                  15

Pro Thr Glu Ser Ile Val Arg Phe Pro Asn Ile Thr Asn Leu Cys Pro
            20                  25                  30

Phe Gly Glu Val Phe Asn Ala Thr Arg Phe Ala Ser Val Tyr Ala Trp
        35                  40                  45

Asn Arg Lys Arg Ile Ser Asn Cys Val Ala Asp Tyr Ser Val Leu Tyr
    50                  55                  60

Asn Ser Ala Ser Phe Ser Thr Phe Lys Cys Tyr Gly Val Ser Pro Thr
65                  70                  75                  80

Lys Leu Asn Asp Leu Cys Phe Thr Asn Val Tyr Ala Asp Ser Phe Val
                85                  90                  95

Ile Arg Gly Asp Glu Val Arg Gln Ile Ala Pro Gly Gln Thr Gly Lys
            100                 105                 110

Ile Ala Asp Tyr Asn Tyr Lys Leu Pro Asp Asp Phe Thr Gly Cys Val
        115                 120                 125

Ile Ala Trp Asn Ser Asn Asn Leu Asp Ser Lys Val Gly Gly Asn Tyr
    130                 135                 140

Asn Tyr Leu Tyr Arg Leu Phe Arg Lys Ser Asn Leu Lys Pro Phe Glu
145                 150                 155                 160

Arg Asp Ile Ser Thr Glu Ile Tyr Gln Ala Gly Ser Thr Pro Cys Asn
                165                 170                 175

Gly Val Glu Gly Phe Asn Cys Tyr Phe Pro Leu Gln Ser Tyr Gly Phe
            180                 185                 190

Gln Pro Thr Asn Gly Val Gly Tyr Gln Pro Tyr Arg Val Val Val Leu
        195                 200                 205

Ser Phe Glu Leu Leu His Ala Pro Ala Thr Val Cys Gly Pro Lys Lys
    210                 215                 220

Ser Thr Asn Leu Val Lys Asn Lys Cys Val Asn Phe Asn Phe
225                 230                 235


<210>   8
<211>   654
<212>   PRT
<213>   Artificial Sequence

<220>
<223>   COVID-19 S1


<400>   8
Gln Cys Val Asn Leu Thr Thr Arg Thr Gln Leu Pro Pro Ala Tyr Thr
1               5                   10                  15

Asn Ser Phe Thr Arg Gly Val Tyr Tyr Pro Asp Lys Val Phe Arg Ser
            20                  25                  30

Ser Val Leu His Ser Thr Gln Asp Leu Phe Leu Pro Phe Phe Ser Asn
        35                  40                  45

Val Thr Trp Phe His Ala Ile His Val Ser Gly Thr Asn Gly Thr Lys
    50                  55                  60

Arg Phe Asp Asn Pro Val Leu Pro Phe Asn Asp Gly Val Tyr Phe Ala
65                  70                  75                  80

Ser Thr Glu Lys Ser Asn Ile Ile Arg Gly Trp Ile Phe Gly Thr Thr
                85                  90                  95

Leu Asp Ser Lys Thr Gln Ser Leu Leu Ile Val Asn Asn Ala Thr Asn

            100                 105                 110

Val Val Ile Lys Val Cys Glu Phe Gln Phe Cys Asn Asp Pro Phe Leu
        115                 120                 125

Gly Val Tyr Tyr His Lys Asn Asn Lys Ser Trp Met Glu Ser Glu Phe
    130                 135                 140

Arg Val Tyr Ser Ser Ala Asn Asn Cys Thr Phe Glu Tyr Val Ser Gln
145                 150                 155                 160

Pro Phe Leu Met Asp Leu Glu Gly Lys Gln Gly Asn Phe Lys Asn Leu
                165                 170                 175

Arg Glu Phe Val Phe Lys Asn Ile Asp Gly Tyr Phe Lys Ile Tyr Ser
            180                 185                 190

Lys His Thr Pro Ile Asn Leu Val Arg Asp Leu Pro Gln Gly Phe Ser
        195                 200                 205

Ala Leu Glu Pro Leu Val Asp Leu Pro Ile Gly Ile Asn Ile Thr Arg
    210                 215                 220

Phe Gln Thr Leu Leu Ala Leu His Arg Ser Tyr Leu Thr Pro Gly Asp
225                 230                 235                 240

Ser Ser Ser Gly Trp Thr Ala Gly Ala Ala Ala Tyr Tyr Val Gly Tyr
                245                 250                 255

Leu Gln Pro Arg Thr Phe Leu Leu Lys Tyr Asn Glu Asn Gly Thr Ile
            260                 265                 270

Thr Asp Ala Val Asp Cys Ala Leu Asp Pro Leu Ser Glu Thr Lys Cys
        275                 280                 285

Thr Leu Lys Ser Phe Thr Val Glu Lys Gly Ile Tyr Gln Thr Ser Asn
    290                 295                 300

Phe Arg Val Gln Pro Thr Glu Ser Ile Val Arg Phe Pro Asn Ile Thr
305                 310                 315                 320

Asn Leu Cys Pro Phe Gly Glu Val Phe Asn Ala Thr Arg Phe Ala Ser
                325                 330                 335

Val Tyr Ala Trp Asn Arg Lys Arg Ile Ser Asn Cys Val Ala Asp Tyr
            340                 345                 350

Ser Val Leu Tyr Asn Ser Ala Ser Phe Ser Thr Phe Lys Cys Tyr Gly
        355                 360                 365

Val Ser Pro Thr Lys Leu Asn Asp Leu Cys Phe Thr Asn Val Tyr Ala
    370                 375                 380

Asp Ser Phe Val Ile Arg Gly Asp Glu Val Arg Gln Ile Ala Pro Gly
385                 390                 395                 400

Gln Thr Gly Lys Ile Ala Asp Tyr Asn Tyr Lys Leu Pro Asp Asp Phe
                405                 410                 415

Thr Gly Cys Val Ile Ala Trp Asn Ser Asn Asn Leu Asp Ser Lys Val
            420                 425                 430

Gly Gly Asn Tyr Asn Tyr Leu Tyr Arg Leu Phe Arg Lys Ser Asn Leu

435                     440                     445

Lys Pro Phe Glu Arg Asp Ile Ser Thr Glu Ile Tyr Gln Ala Gly Ser
    450                     455                     460

Thr Pro Cys Asn Gly Val Glu Gly Phe Asn Cys Tyr Phe Pro Leu Gln
465                     470                     475                     480

Ser Tyr Gly Phe Gln Pro Thr Asn Gly Val Gly Tyr Gln Pro Tyr Arg
                485                     490                     495

Val Val Val Leu Ser Phe Glu Leu Leu His Ala Pro Ala Thr Val Cys
            500                     505                     510

Gly Pro Lys Lys Ser Thr Asn Leu Val Lys Asn Lys Cys Val Asn Phe
        515                     520                     525

Asn Phe Asn Gly Leu Thr Gly Thr Gly Val Leu Thr Glu Ser Asn Lys
    530                     535                     540

Lys Phe Leu Pro Phe Gln Gln Phe Gly Arg Asp Ile Ala Asp Thr Thr
545                     550                     555                     560

Asp Ala Val Arg Asp Pro Gln Thr Leu Glu Ile Leu Asp Ile Thr Pro
                565                     570                     575

Cys Ser Phe Gly Gly Val Ser Val Ile Thr Pro Gly Thr Asn Thr Ser
            580                     585                     590

Asn Gln Val Ala Val Leu Tyr Gln Asp Val Asn Cys Thr Glu Val Pro
        595                     600                     605

Val Ala Ile His Ala Asp Gln Leu Thr Pro Thr Trp Arg Val Tyr Ser
    610                     615                     620

Thr Gly Ser Asn Val Phe Gln Thr Arg Ala Gly Cys Leu Ile Gly Ala
625                     630                     635                     640

Glu His Val Asn Asn Ser Tyr Glu Cys Asp Ile Pro Ile Gly
                645                     650


<210>   9
<211>   103
<212>   PRT
<213>   Artificial Sequence

<220>
<223>   Heat-labile toxin B subunit(LTB)


<400>   9
Ala Pro Gln Thr Ile Thr Glu Leu Cys Ser Glu Tyr Arg Asn Thr Gln
1               5                   10                  15

Ile Tyr Thr Ile Asn Asp Lys Ile Leu Ser Tyr Thr Glu Ser Met Ala
            20                  25                  30

Gly Lys Arg Glu Met Val Ile Ile Thr Phe Lys Ser Gly Glu Thr Phe
        35                  40                  45

Gln Val Glu Val Pro Gly Ser Gln His Ile Asp Ser Gln Lys Lys Ala
    50                  55                  60

Ile Glu Arg Met Lys Asp Thr Leu Arg Ile Thr Tyr Leu Thr Glu Thr
65                  70                  75                  80

Lys Ile Asp Lys Leu Cys Val Trp Asn Asn Lys Thr Pro Asn Ser Ile
                85                  90                  95

Ala Ala Ile Ser Met Lys Asn
            100

<210>    10
<211>    158
<212>    PRT
<213>    Artificial Sequence

<220>
<223>    Bacterioferritin

<400>    10
Met Lys Gly Asp Thr Lys Val Ile Asn Tyr Leu Asn Lys Leu Leu Gly
1               5                   10                  15

Asn Glu Leu Val Ala Ile Asn Gln Tyr Phe Leu His Ala Arg Met Phe
            20                  25                  30

Lys Asn Trp Gly Leu Lys Arg Leu Asn Asp Val Glu Tyr His Glu Ser
        35                  40                  45

Ile Asp Glu Met Lys His Ala Asp Arg Tyr Ile Glu Arg Ile Leu Phe
    50                  55                  60

Leu Glu Gly Leu Pro Asn Leu Gln Asp Leu Gly Lys Leu Asn Ile Gly
65                  70                  75                  80

Glu Asp Val Glu Glu Met Leu Arg Ser Asp Leu Ala Leu Glu Leu Asp
                85                  90                  95

Gly Ala Lys Asn Leu Arg Glu Ala Ile Gly Tyr Ala Asp Ser Val His
            100                 105                 110

Asp Tyr Val Ser Arg Asp Met Met Ile Glu Ile Leu Arg Asp Glu Glu
        115                 120                 125

Gly His Ile Asp Trp Leu Glu Thr Glu Leu Asp Leu Ile Gln Lys Met
    130                 135                 140

Gly Leu Gln Asn Tyr Leu Gln Ala Gln Ile Arg Glu Glu Gly
145                 150                 155

<210>    11
<211>    471
<212>    PRT
<213>    Artificial Sequence

<220>
<223>    RSV F

<400>    11
Gln Asn Ile Thr Glu Glu Phe Tyr Gln Ser Thr Cys Ser Ala Val Ser

1               5                   10                  15

Lys Gly Tyr Leu Ser Ala Leu Arg Thr Gly Trp Tyr Thr Ser Val Ile
            20                  25                  30

Thr Ile Glu Leu Ser Asn Ile Lys Glu Asn Lys Cys Asn Gly Thr Asp
        35                  40                  45

Ala Lys Val Lys Leu Ile Lys Gln Glu Leu Asp Lys Tyr Lys Asn Ala
    50                  55                  60

Val Thr Glu Leu Gln Leu Leu Met Gln Ser Thr Pro Pro Thr Asn Asn
65                  70                  75                  80

Arg Ala Arg Arg Gly Gly Ser Gly Gly Ser Gly Gly Ser Gly Phe Leu
                85                  90                  95

Gly Phe Leu Leu Gly Val Gly Ser Ala Ile Ala Ser Gly Val Ala Val
            100                 105                 110

Cys Lys Val Leu His Leu Glu Gly Glu Val Asn Lys Ile Lys Ser Ala
        115                 120                 125

Leu Leu Ser Thr Asn Lys Ala Val Val Ser Leu Ser Asn Gly Val Ser
    130                 135                 140

Val Leu Thr Phe Lys Val Leu Asp Leu Lys Asn Tyr Ile Asp Lys Gln
145                 150                 155                 160

Leu Leu Pro Ile Leu Asn Lys Gln Ser Cys Ser Ile Ser Asn Ile Glu
                165                 170                 175

Thr Val Ile Glu Phe Gln Gln Lys Asn Asn Arg Leu Leu Glu Ile Thr
            180                 185                 190

Arg Glu Phe Ser Val Asn Ala Gly Val Thr Thr Pro Val Ser Thr Tyr
        195                 200                 205

Met Leu Thr Asn Ser Glu Leu Leu Ser Leu Ile Asn Asp Met Pro Ile
    210                 215                 220

Thr Asn Asp Gln Lys Lys Leu Met Ser Asn Asn Val Gln Ile Val Arg
225                 230                 235                 240

Gln Gln Ser Tyr Ser Ile Met Cys Ile Ile Lys Glu Glu Val Leu Ala
                245                 250                 255

Tyr Val Val Gln Leu Pro Leu Tyr Gly Val Ile Asp Thr Pro Cys Trp
            260                 265                 270

Lys Leu His Thr Ser Pro Leu Cys Thr Thr Asn Thr Lys Glu Gly Ser
        275                 280                 285

Asn Ile Cys Leu Thr Arg Thr Asp Arg Gly Trp Tyr Cys Asp Asn Ala
    290                 295                 300

Gly Ser Val Ser Phe Phe Pro Gln Ala Glu Thr Cys Lys Val Gln Ser
305                 310                 315                 320

Asn Arg Val Phe Cys Asp Thr Met Asn Ser Leu Thr Leu Pro Ser Glu
                325                 330                 335

Ile Asn Leu Cys Asn Val Asp Ile Phe Asn Pro Lys Tyr Asp Cys Lys

340                 345                 350

Ile Met Thr Ser Lys Thr Asp Val Ser Ser Ser Val Ile Thr Ser Leu
        355                 360                 365

Gly Ala Ile Val Ser Cys Tyr Gly Lys Thr Lys Cys Thr Ala Ser Asn
    370                 375                 380

Lys Asn Arg Gly Ile Ile Lys Thr Phe Ser Asn Gly Cys Asp Tyr Val
385                 390                 395                 400

Ser Asn Lys Gly Met Asp Thr Val Ser Val Gly Asn Thr Leu Tyr Tyr
                405                 410                 415

Val Asn Lys Gln Glu Gly Lys Ser Leu Tyr Val Lys Gly Glu Pro Ile
            420                 425                 430

Ile Asn Phe Tyr Asp Pro Leu Val Phe Pro Ser Asp Glu Phe Asp Ala
        435                 440                 445

Ser Ile Ser Gln Val Asn Glu Lys Ile Asn Gln Ser Leu Ala Phe Ile
    450                 455                 460

Arg Lys Ser Asp Glu Leu Leu
465                 470


<210>    12
<211>    104
<212>    PRT
<213>    Artificial Sequence

<220>
<223>    JEV Envelope domain III(ED3)


<400>    12
Lys Gly Thr Thr Tyr Gly Met Cys Thr Glu Lys Phe Ser Phe Ala Lys
 1               5                  10                  15

Asn Pro Ala Asp Thr Gly His Gly Thr Val Val Ile Glu Leu Ser Tyr
            20                  25                  30

Ser Gly Ser Asp Gly Pro Cys Lys Ile Pro Ile Val Ser Val Ala Ser
        35                  40                  45

Leu Asn Asp Met Thr Pro Val Gly Arg Leu Val Thr Val Asn Pro Phe
    50                  55                  60

Val Ala Thr Ser Ser Ala Asn Ser Lys Val Leu Val Glu Met Glu Pro
65                  70                  75                  80

Pro Phe Gly Asp Ser Tyr Ile Val Val Gly Arg Gly Asp Lys Gln Ile
                85                  90                  95

Asn His His Trp His Lys Ala Gly
            100

## Claims

1. A peptide for enhancing the expression efficiency of a target protein, comprising the sequence of a domain isolated from human glutamyl-prolyl tRNA synthetase (hEPRS).

2. The peptide of claim 1, wherein the domain includes TRS-1 (SEQ ID NO: 1) and TRS-2 (SEQ ID NO: 2).

3. The peptide of claim 2, wherein the domain further includes TRS-3 (SEQ ID NO: 3).

4. The peptide of claim 1, further comprising linkers for connecting the sequences of the domains.

5. The peptide of claim 4, wherein the linker sequence is represented by SEQ ID NO: 4 or 5.

6. The peptide of claim 1, further comprising a tag sequence for improving the expression efficiency of a target protein.

7. The peptide of claim 1, wherein the target protein includes one or more selected from the group consisting of an antigen, an antibody, a cell receptor, an enzyme, a structural protein, serum, and a cell protein.

8. The peptide of claim 7, wherein the antigen includes one or more selected from the group consisting of novel coronavirus (SARS-CoV-2), the hemagglutinin (HA) of influenza virus, respiratory syncytial virus (RSV), and Nipah virus.

9. A polynucleotide encoding the peptide according to any one of claims 1 to 8.

10. An expression vector, comprising:
a polynucleotide encoding a target protein; and
a polynucleotide encoding a peptide according to any one of claims 1 to 8 binding to the 5'-end of the polynucleotide encoding the target protein.

11. A host cell transformed with the expression vector of claim 10.

12. The host cell of claim 11, which is *E. coli.*

13. A method of producing a soluble target protein, comprising:
(A) preparing an expression vector including a polynucleotide encoding a target protein and a nucleotide encoding the peptide of any one of claims 1 to 8 binding to the 5' end of the polynucleotide;
(B) preparing a transformant by introducing the expression vector into host cells; and
(C) culturing the transformant to induce the expression of a recombinant target protein and obtaining the target protein.
